Europäisches Patentamt

European Patent Office

Office européen des brevets

(11) Publication number: **0 621 540 A1**

# EUROPEAN PATENT APPLICATION

(21) Application number: **93201141.4**

(51) Int. Cl.5: **G06F 15/20**

(22) Date of filing: **19.04.93**

(43) Date of publication of application:
**26.10.94 Bulletin 94/43**

(84) Designated Contracting States:
**DE FR GB**

(71) Applicant: **Babloyantz, Agnessa**

Avenue Blucher 199
B-1180 Bruxelles (BE)

(72) Inventor: **Babloyantz, Agnessa**
Avenue Blucher 199
B-1180 Bruxelles (BE)

(54) **Graphical representation of local relationships in a data set.**

(57) A graphical method is devised whereby the local relationships called hereafter "correlations" in a data set are represented in two, three or n dimensional space. The two dimensional first order diagrams correlate three successive events. The difference between the second and the first event is the x coordinate and the difference between the third and the second is the y cartesian coordinate. The two dimensional second order diagrams are obtained by considering four events. The y coordinate is the sum of the fourth and the second minus two times the third event whereas the x coordinates is the sum of the third and the first minus two times the second event. The diagrams could be generalized to n order and m spatial dimensions in any system of coordinate thus extending the local domain under consideration. If local correlations exist in the data set, the diagrams of all orders show well defined structures. Each part of a structure can be related to well defined events characterizing the processes under investigation. This invention is particularly important in cardiology. It detects in a single glance, quantitatively and qualitatively, the arrhythmias and the fluctuations of cardiac activity thus in general probes cardiac dynamics. This invention is a powerful tool for detection of existing pathological activity, monitoring the effect of drugs, assessing the change in cardiac dynamics, due to aging and detecting the onset of states leading to heart failure before the catastrophic event. The conceptual simplicity of the method, which could be implemented on a personal computer makes this invention a valuable tool for cardiologists and also for the general practitioners. The invention could be applied to any situation where correlations are present in a long succession of sequential events or measured quantities, such as for example in electrophysiology.

Figure 4

Figure 6

EP 0 621 540 A1

## Background

The present invention relates generally to sequence analysis and, more particularly, is designed to assess local relationships, hereafter conveniently called "correlations", in an ordered sequence of events and in a geometrical representation. The geometrical representations of local correlations are of common use for assessing the presence of chaotic dynamics in physical, chemical and biological systems as well as cardiac activity. Reference is hereby made for examples to J. Guckenheimer and P. Holmes (in Nonlinear oscillations, dynamical systems and bifurcations of vector fields. Springer-Verlag)

So called " return maps " display, in a two dimensional system, an element of the sequence as a function of the preceding element. A well defined structure in this display indicates the existence of local correlations. However in most systems of practical importance these short range correlations do not furnish structured, i.e. exploitable patterns. The elaboration of a method for assessing local correlations over several consecutive events may furnish valuable information about the underlying mechanism which generates the correlations. A geometrical display of such correlations may become a powerful tool in such fields as cardiology and neurophysiology.

An attempt has been made by Woo , Stevenson , Moser, Trelease and Harper (Patterns of beat-to-beat heart rate variability in advanced heart failure. American Heart Journal Vol 123 No 3 704-710 March 1992) to parallel by visual inspection, the shape of return maps with cardiac pathologies. Such an endeavour does not furnish any qualitative or quantitative conclusions about the underlying pathologies. With the present invention we extend the assessment of local correlations between elements of a data set to three, four , five or n consecutive elements. Whenever such correlations exist, geometrical representations in two, three and m dimensions show well defined structures. The latter could be given precise meaning and quantitative assessment with regard to the underlying process that generated the data set.

The field of cardiology is one of the most suitable domains of application of our invention. The automatic assessment over periods up to 24 hour of cardiac activity can be performed and the result displayed in a simple graph. Arrhythmias of all nature appear as well defined structures that in some cases can be cast into mathematical equations.

The analysis of the graph does not require advanced mathematical knowledge. A simple software can be implemented on a personal computer in any doctor's office. Thus the characterization of all types of arrhythmias, the change of cardiac dynamics as a result of aging and drug consumption could be monitored qualitatively as well as quantitatively and with ease. Thus this invention provides an inexpensive and user friendly tool for cardiologists as well as for the general practitioners.

## Description of the Invention

Consider a long series of discrete events or measures in succession, obtained from a process, hereafter called a data set. The difference between two successive events $r_i$ and $r_{i+1}$ is not necessarily constant and may seem random. It is important to discover whether the succession of the $r_i$'s considered as a data set is totally random or if correlations exist between the intervals. If such correlations can be demonstrated, important information about the underlying process or dynamics may be obtained. The unveiling of these correlations may have potential applications in certain circumstances as will become clear in the sequel.

Any event $r_i$ can be related to the next event in a two dimensional diagram which is known as the first return map. One plots $r_{i+1}$ as a function of $r_i$ and this for all events of the data set. If there is an obvious correlation between the $r_i$'s, a recognizable pattern will emerge. In the absence of correlations a cloud of points is seen. In this procedure local correlations between two successive points are represented geometrically. Unfortunately, unless one is in the presence of, for example, a low dimensional chaotic dynamics, usually the return maps do not furnish usable information.

However the procedure of mapping local correlations into geometrical representations could be generalized to correlations between three, four, five or higher number of events. Thus the geometrical representation could be either in a two dimensional plane or in three dimensional space or more, generally in m dimensional space. Several types of diagrams will be considered.

A *first order diagram* which correlates three successive events is constructed in the following manner ; by taking the difference between two successive intervals a new data set is obtained. From this data set a two dimensional first order diagram is constructed by taking $r'_i = r_i - r_{i-1}$ and $r'_{i+1} = r_{i+1} - r_i$ as the coordinates of a point in a system of cartesian coordinates.

If correlations exist in this data set, they appear as well defined geometrical objects. The latter can be characterised quantitatively and qualitatively and may be related to given processes. Thus a knowledge can be gained about the behaviour of the underlying system which has generated the data set. This point will be illustrated with the application of the procedure to the specific case of heart rate variability. The elementary software can be implemented on a personal computer.

A similar diagram may be constructed in three dimensional space where a new axis $r'_{i+2} = r_{i+2} - r_{i+1}$ is necessary. The three dimensional diagram of first order thus correlates four successive events.

The quantification of three dimensional diagrams necessitates special software. Higher dimensional diagrams may also be constructed by similar procedures and can be exploited by the use of appropriate software.

A more practical way to represent correlations between four successive events could be devised in the following manner. As for the case of the first order diagrams we define a new data set where $r''_i = r'_i - r'_{i-1}$ ; $r''_{i+1} = r'_{i+1} - r'_i$ . A two dimensional diagram constructed from these quantities, in the manner of a first order diagram, defines a *second order diagram*.

If, as in the first order diagrams, local correlations exist among consecutive events, well defined geometrical structures also appear in the second order diagrams and may be characterised qualitatively and quantitatively.

The second order diagram can also be represented in three or higher dimensions. Let us keep in mind that in three dimensional second order diagrams the relationship between the original $r_i$'s and the three coordinate axis is as follows :

$$
\begin{aligned}
x_i &= r_i - 2\,r_{i-1} + r_{i-2} \\
y_i &= r_{i+1} - 2\,r_i + r_{i-1} \\
z_i &= r_{i+2} - 2r_{i+1} + r_i
\end{aligned}
\tag{1}
$$

In two dimensions the second order diagram correlates four events as it is the case in three dimensions and first order diagrams. However let us note that the quantities which are plotted are different in two dimensional second order and three dimensional first order diagrams. In the former case one is in the presence of information compression.

The procedure can be generalized to nth order diagrams in m dimensional space. Let us note however, as the order of the diagrams increases and space dimension is kept low, one sees an information compression.

So far the diagrams of the first and second order were expressed in cartesian coordinates. However if special symmetries are detected in this system of coordinates the passage to other system of coordinates such as polar or hyperbolic coordinates may be of value.

The diagrams obtained above are ideal for probing electrical activity of the heart. The latter is measured non invasively with the help of electrodes which lie on the skin. In normal subjects these so called electrocardiograms (ECG) have a characteristic shape as depicted in Fig.(1) and repeat themselves. The basic pattern is decomposed in P and T waves and in QRS complexes.

Even in normal cardiac activity, the time intervals between the different waves (RR, PR, PQ, ST, RT etc...) are not constant and may vary in certain limits from one beat to the next. The variability of RR intervals results from sinus arrhythmias and is influenced by respiratory function. All the above mentioned distances are related to given physiological states of cardiac activity.

In phatologies, the variations of these distances may become considerable and one speaks of abnormal arrhythmias thus indicating the presence of functional or structural changes of the heart. In some instances considerable deformations of electrocardiogram are seen which may slightly change the various important intervals of Fig.(1). Such changes are also detected by our method.

The sinusal arrhythmia is related to the activity of the primary pacemaker of the heart: the sinusal node. Other arrhythmias may originate from other locations in the atria. These are supraventricular ectopic arrhythmias: extrasystoles, atrial flutter or atrial fibrillation. They all involve a more or less severe alteration of the basic normal rhythm.

Moreover, all deformation of the morphology of the P, T waves and QRS complex introduces a change in all PQ, ST, QT distances. Thus all the relevant distances in the electrocardiogram could be considered as a succession of events $r_i$ ordered in time and are suitable for construction of diagrams of first, second and nth order in two, three and m dimensions.

If all above mentioned arrhythmias arise randomly in time, a shapeless cloud like structure is expected for all diagrams of all order and in any system of coordinates. However if the occurrence of abnormal activity of any nature follows a given deterministic rule, at least for a short time, but repeated over long periods, then well defined structures and shapes could be seen in all diagrams. Moreover one could relate

EP 0 621 540 A1

each structure to a well defined physiological event and eventually to a simple rule.

For the sake of simplicity we limit the discussion to first order and second order diagrams in two dimensions and we limit ourself to the study of RR intervals. The third dimensional diagrams are best visualized on computer screens. The power of the method will be illustrated with examples of normal and pathological cardiac activity.

It is well known that in healthy individuals the normal sinus rhythm is under the influence of respiratory system which imprints some degree of variability on the pacemaker activity. Fig.(2.a) and (2.b) shows the values of RR intervals recorded from two healthy young subjects. In Fig.(3.a) and (3.b) the corresponding first order diagrams are shown together with one constructed from random noise (Fig.(3.c) ). One sees immediately the presence of correlations in the healthy cardiac activity whereas the random noise is unstructured. The shape of Fig.(3.c) is due to the uniform probability distribution which is non-zero on a well defined interval. A moments reflexion shows that if three successive RR or interbeat intervals in Fig.(2.a) are the same, then $r_i - r_{i-1} = 0$ , $r_{i+1} - r_i = 0$ , and we have the trivial point (0,0). On the other hand the quadrant I is the locus of all triplets of intervals such that the distances increase in each step indicating a deceleration of the cardiac activity. On the contrary when three successive intervals decrease at each step, a point appears in the quadrant III indicating a gradual acceleration of the heart. The quadrants II and IV represent respectively points such as short-long-short and long-short-long triplets are detected.

By comparing Fig.(3.a) and (3.b) one sees immediately the power of the diagrams which are able to distinguish between two sinus arrhythmias from two normal cardiac activities. A more interesting first order diagram is shown on Fig.(4) where the ECG of the individual comprises premature beats or extrasystoles which may be of different type according to their distance to the next normal beat. One sees obvious structures that can be analysed separately and which indicate the presence of correlations between three successive interbeat intervals.

As it is said already, in this diagram three successive and identical RR correspond to $x = 0$, $y = 0$. Therefore all points in the small circle around the origin may be considered as emerging from triplets of almost equal intervals, the difference between the intervals being of the order of the radius of the circles.

Now three almost equal RR in succession of any size will generate a point in this small circle. In order to obtain a better resolution we construct the first return map as described before however only with these selected points. Such a return map is shown on Fig.(5) where a cigar shaped structure is seen. The coordinates of the points P and M, the boundaries of the cigar, indicate respectively the minimum and the maximum interval within triplets of this type. The longer the cigar, the more distributed are the intervals.

A more formal description of laws governing the correlations may be found in the following manner. The axis (1) on Fig.(4) is the locus of triplets with a well defined relationship. The axis (1) is described by the linear equation $y = -x$ and $x < 0$ which requires that for all interval length

$$y = r_{i+1} - r_i = - (r_i - r_{i-1}) = -x \qquad (2)$$

thus

$$r_{i+1} = r_{i-1} > r_i$$

Thus all triplets obeying to Eq. (2) contribute to a point on axis (1). Although triplet lengths are different, a smaller RR is surrounded by two large and equal intervals. Similar reasoning applies to the axis (2). However in this case we have the relation

$$r_{i+1} = r_{i-1} < r_i \qquad (3)$$

The axis (3) on Fig.(4), not very pronounced but still visible, is described by the equation $y = - \alpha x + \beta$ ( $\alpha$ and $\beta$ are positive constants). Here $\beta$ is the value of y at the intersection of this axis with the axis $x = 0$, while $\alpha$ is the slope of the axis. Both values can easily be estimated from the graph.

In terms of RR intervals this equation implies the following relations between the three intervals.

$$r_{i+1} - r_i = - \alpha (r_i - r_{i-1}) + \beta \qquad (4)$$

or

$$r_{i+1} = r_i (1-\alpha) + \alpha r_{i-1} + \beta$$

4

The axis (3) suggests that two types of triplets $r_{i+1} > r_i > r_{i-1}$ or $r_{i+1} > r_i$ , $r_{i-1} > r_i$ are ordered in such a way that the equation (4) is satisfied. Similar reasoning applies to axis (4) and one sees that the triplets $r_{i-1}$, $r_i$ , $r_{i+1}$ again obey to a linear equation.

The linear part of axis (5) obeys to the equation $y = -\alpha' x$ which implies the following relations between the triplet of intervals :

$$r_{i+1} - r_i = -\alpha' ( r_i - r_{i-1} ) \qquad (5)$$

Again, the slope $\alpha'$ can be estimated from the graph. Dimilar reasoning applies to axis(6).

Time does not appear explicitly in these diagrams. However the timing of events in a given pattern or a portion of a pattern can be assessed in the following manner. One chooses a window comprising a given pattern or some part of it. With the help of a simple algorithm one extracts from the total data set all triplets that produce a point in the window. For each triplet one collects the exact value of the three intervals and the time at which they occur.

Fig.(6) shows a second order diagram from the same patient. One sees again the appearance of well defined structures.

With the same type of reasoning, as in the case of first order diagrams, relationships between four distances giving rise to second order diagrams, in two dimensions, could be found. It is also possible to express the equations describing various shapes lying on these diagrams in terms of RR intervals. In the present case these relationships are much more involved and will not be given here.

In high order diagrams in three or higher space dimensions the equations describing the patterns involve too many terms.

Fig.(7) shows the first order diagrams of two cardiac pathologies. Fig.(7.a) is recorded from a patient suffering from lung and cardiac deficiency, Fig.(7.b) is a case of atrial fibrillation. These examples demonstrate the power and the usefulness of our invention.

In order to see the significance of first and second order patterns in the field of cardiology, we construct artificial sequences of intervals reproducing the different kinds of known extrasystoles and we plot the first and second order diagrams for each of them. Fig.(8) shows six first order diagrams of two types of extrasystoles. Namely extrasystoles with compensatory pause (Fig.(8.a-c) ) and interpolated extrasystoles (Fig.(8.d-f) ). For each of these types we consider three cases: isolated, in the form of bigeminy and in the form of trigeminy.

These "theoretical" diagrams can be used as a catalogue since by construction the nature of each pattern is well defined. The first order patterns obtained from electrocardiograms could thus be compared with this catalogue and corresponding events be identified. A comparison between Fig.(4) and Fig.(8) shows that we are in the presence of extrasystoles with compensatory pause which occur isolated (Fig.(8.a))or in the form of bigeminy (Fig.(8.b)) , but also to a lesser extent interpolated extrasystoles are seen (Fig.(8.d)).

Fig.(9) shows six theoretical diagrams of second order constructed from the artificial set of RR intervals. They correspond to the same cases as in Fig.(8).

The reading of the diagrams of all order can be made more quantitative by colour or gray level coding of density of points on each pattern.

The analysis of correlations in EEG or in neural firing could also be performed profitably with the help of this invention.

The invention has now been described with reference to specific embodiments. Other embodiments will be apparent to those of ordinary skill in the art. For example the invention herein described applies to any situation where correlations between successive events, measures or values can be demonstrated. It is therefore, not intended that the invention be limited to the appended claims.

**Brief description of the drawings**

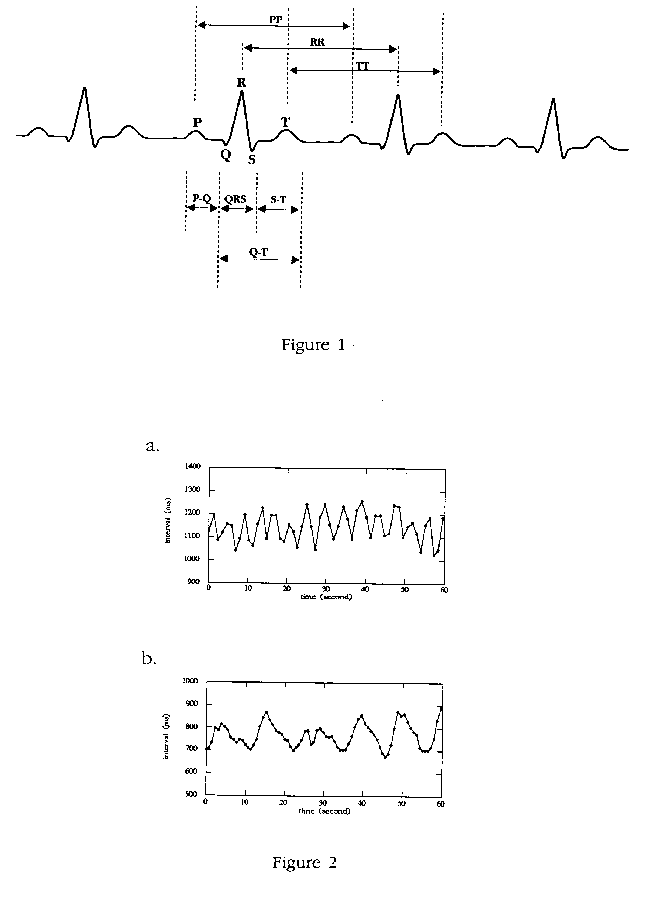

Fig.1.     Various components of a normal electrocardiogram

Fig.2.     Values of cardiac interbeat intervals of a healthy subject as a function of time

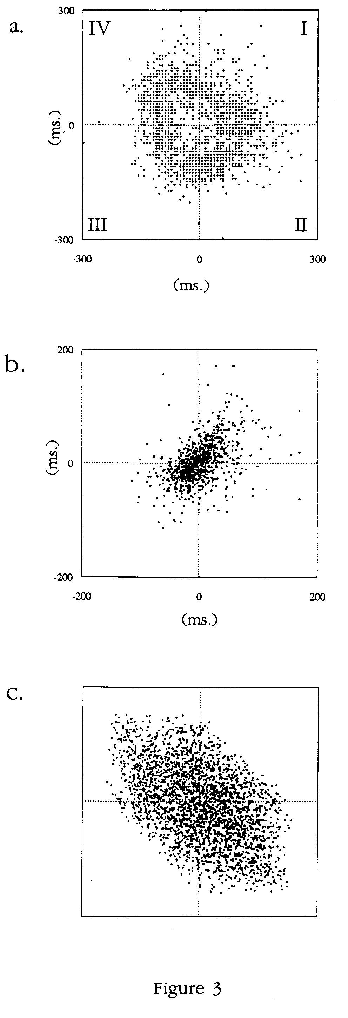

Fig.3.     First order diagrams of two healthy subjects (a-b) and a of a randoml distribution of events with uniform probability (c)

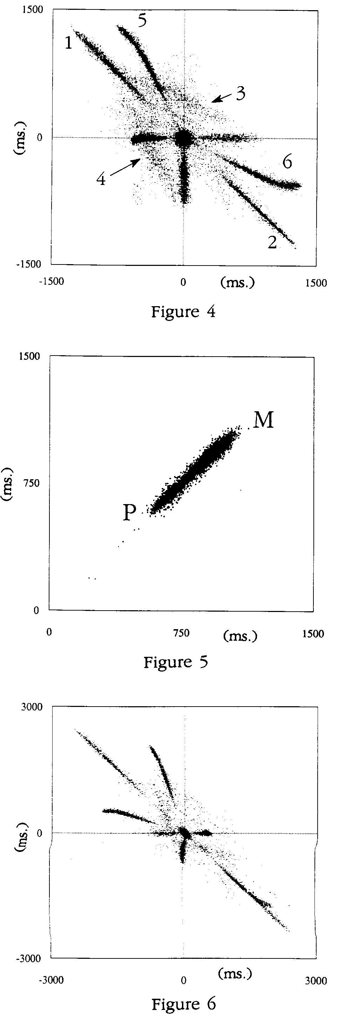

Fig. 4.     First order diagram of a patient with extrasystoles

Fig.5.     A return map of almost equal triplets extracted from a first order diagram

Fig.6.     Second order diagram of a patient with extrasystoles (same patient as in Fig.4.)

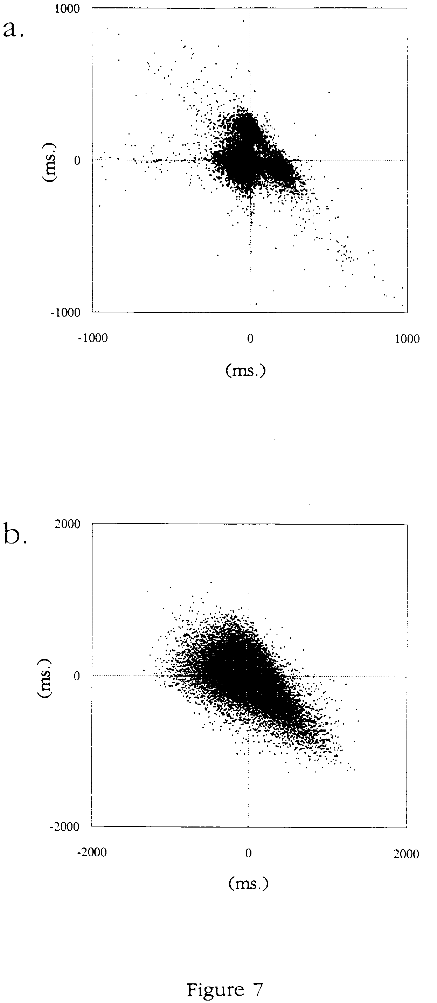

Fig.7.     First order diagrams of patients suffering from (a) lung and cardiac deficiency, (b) atrial fibrillation.

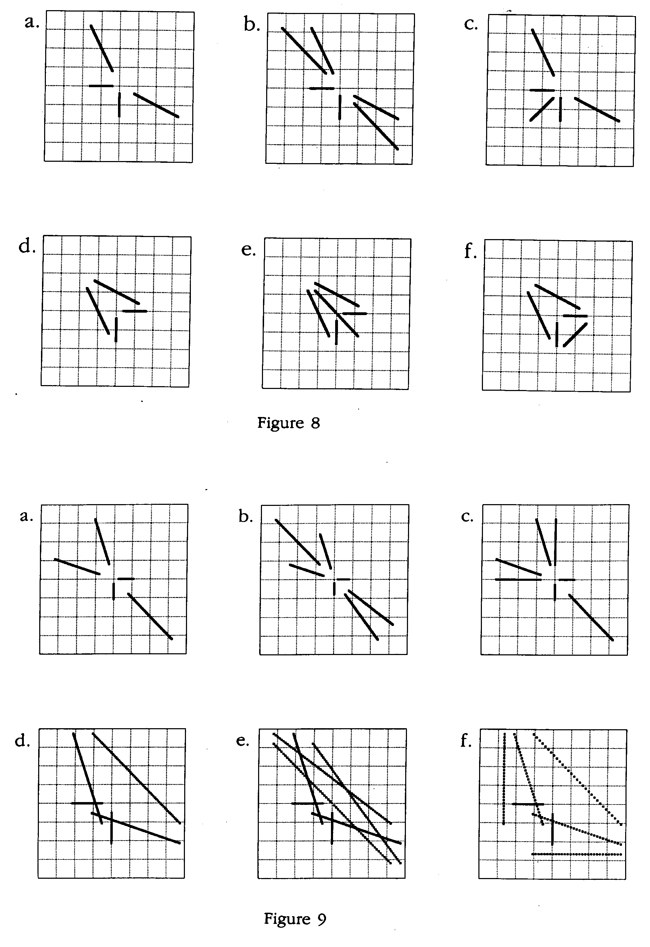

Fig.8.  First order diagrams :
for extrasystoles with a compensatory pause : (a) isolated (b) bigeminy (c) trigeminy, for interpolated extrasystoles : (d) isolated (e) bigeminy (f) trigeminy

Fig.9.  Second order diagrams :
for extrasystoles with a compensatory pause : (a) isolated (b) bigeminy (c) trigeminy, for interpolated extrasystoles : (d) isolated (e) bigeminy (f) trigeminy

**Claims**

1. A method to analyse and graphically represent local correlations among time intervals $r_i$ between successive events in a sequence of events, where the ensemble of time intervals $r_i$ is called a data set, characterized by the construction of n-th order diagrams in m-dimensional space, where $n = 1, 2, 3, ...$ and $m = 2, 3, ...$, as follows :

take $n + m$ successive intervals $r_{i-n}, r_{i-n+1}, ..., r_i, ..., r_{i+m-1}$ from the data set ; calculate the components

$$x_i^{(1)}, x_i^{(2)}, ..., x_i^{(m)}$$

defined by

$$x_i^{(1)} = \sum_{k=0}^{n} (-1)^k \binom{n}{k} r_{i-k}$$

$$x_i^{(2)} = \sum_{k=0}^{n} (-1)^k \binom{n}{k} r_{i-k+1}$$

$$x_i^{(m)} = \sum_{k=0}^{n} (-1)^k \binom{n}{k} r_{i-k+m-1}$$

with

$$\binom{n}{k} = \frac{n!}{k! \, (n-k)!}$$

plot the point

$$( x_i^{(1)}, x_i^{(2)}, ..., x_i^{(m)} )$$

in a

$$x_i^{(1)}, x_i^{(2)}, ..., x_i^{(m)}$$

6

- coordinate system calculate and plot the next and the following points analogously by a one unit shift ($i \rightarrow i+1$), in the data set.

2. A method according to claim 1 characterized by the construction of a first order diagram ($n=1$) in a two dimensional space ($m=2$) as follows :
take three successive intervals $r_{i-1}$ , $r_i$, $r_{i+1}$ from the data set ;
calculate the component $x_i$, $y_i$ defined by :

$x_i = r_i - r_{i-1}$
$y_i = r_{i+1} - r_i$

plot the point ( $x_i$ , $y_i$ ) in a cartesian x, y - coordinate system ;
calculate and plot the next and the following points analogously by a one unit shift ($i \rightarrow i+1$) in the data set.

3. A method according to claim 1 characterized by the construction of a first order diagram ($n=1$) in three dimensional space ($m=3$) as follows:
take four successive intervals $r_{i-1}$ , $r_i$, $r_{i+1}$ , $r_{i+2}$ from the data set ;
calculate the components $x_i$, $y_i$ $z_i$ defined by :

$x_i = r_i - r_{i-1}$
$y_i = r_{i+1} - r_i$
$z_i = r_{i+2} - r_{i+1}$

plot the point ( $x_i$, $y_i$ , $z_i$ ) in a cartesian x, y, z - coordinate system ;
calculate and plot the next and the following points analogously by a one unit shift ($i \rightarrow i+1$) in the data set.

4. A method according to claim 1 characterized by the construction of a second order diagram ($n=2$) in two dimensional space ($m=2$) as follows:
take four successive intervals $r_{i-2}$, $r_{i-1}$, $r_i$, $r_{i+1}$ from the data set ;
calculate the components $x_i$, $y_i$ defined by :

$x_i = r_i - 2 \, r_{i-1} + r_{i-2}$
$y_i = r_{i+1} - 2 \, r_i + r_{i-1}$

plot the point ( $x_i$ , $y_i$) in a cartesian x , y - coordinate system ;
calculate and plot the next and the following points analogously by a one unit shift ($i \rightarrow i+1$) in the data set.

5. A method according to claim 1 characterized by the construction of a second order diagram ($n=2$) in three dimensional space ($m=3$) as follows:
take five successive intervals $r_{i-2}$ , $r_{i-1}$ , $r_i$, $r_{i+1}$ , $r_{i+2}$ from the data set ;
calculate the components $x_i$, $y_i$ $z_i$ defined by :

$x_i = r_i - 2 \, r_{i-1} + r_{i-2}$
$y_i = r_{i+1} - 2 \, r_i + r_{i-1}$
$z_i = r_{i+2} - 2 \, r_{i+1} + r_i$

plot the point ( $x_i$ , $y_i$ , $z_i$) in a cartesian x, y, z - coordinate system ;
calculate and plot the next and the following points analogously by a one unit shift ($i \rightarrow i+1$) in the data set.

6. A method according to claims 1-5, wherein the cartesian coordinates are transformed into polar or hyperbolic coordinates.

7. A method according to claims 1-6 whereafter only events around the origin of the coordinate system are isolated and displayed on a return map as follows :

EP 0 621 540 A1

take two successive intervals $r_i$, $r_{i+1}$ ;
calculate

$$x_i = r_i$$
$$y_i = r_{i+1}$$

plot the point $(x_i, y_i)$ in a cartesian x , y - coordinate system ;
calculate and plot the next and the following points analogously by a one unit shift ($i \rightarrow i+1$) in the data set.

8. Application of the method according to claims 1-7 to the monitoring and to the analysis of the electrical activity of the heart wherein the data set is composed of the relevant intervals (RR, TT, PT, QR or other intervals) between events as they appear in an electrocardiogram.

9. Application according to claim 8 wherein the characteristic patterns or shapes of a specific diagram, which is constructed from a data set related to a specific pathological cardiac activity, is classified according to this specific pathological cardiac activity.

10. Application of the methods 1-9 wherein the density of points in a given diagram is displayed by colour coding or density evaluation.

8

Figure 1

a.

b.

Figure 2

Figure 3

Figure 4

Figure 5

Figure 6

a.

b.

Figure 7

a.

b.

c.

d.

e.

f.

Figure 8

a.

b.

c.

d.

e.

f.

Figure 9

European Patent Office

EUROPEAN SEARCH REPORT

Application Number

EP 93 20 1141
Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | PROCEEDINGS OF COMPUTERS IN CARDIOLOGY, IEEE COMPUTER SOCIETY PRESS NEW YORK US 7 October 1986, BOSTON MASSACHUSETTS US pages 439 - 442 D.T.KAPLAN ET AL 'APPLICATION OF NON LINEAR DYNAMICS TO THE CHARACTERIZATION OF CARDIAC ELECTRICAL INSTABILITY' * page 439, left column, line 7 - line 13 * | 1,2,3,8, 9,10 | G06F15/20 |
| Y | * page 440, left column, line 51 - right column, line 10 * | 4-7 | |
| A | PROGRESS OF THEORETICAL PHYSICS SUPPLEMENT no. 98, 1989, IAPAN pages 400 - 419 HITOSHI YAMAZAKI ET AL 'CHAOS IN SPIN-WAVE INSTABILITIES' * page 410, line 19 - line 28 * | 1-3 | |
| Y | * page 410, line 40 - page 411, line 2 * * page 412, line 5 - line 12 * * page 413, line 16 - line 21 * * page 416, line 15 - line 18 * | 4-7 | |
| | | | **TECHNICAL FIELDS SEARCHED (Int. Cl.5)** |
| X | COMPUTERS IN BIOLOGY AND MEDICINE, PERGAMON PRESS vol. 21, no. 4, 1991, UK pages 243 - 264 T.A.DENTON ET AL 'CAN THE ANALYTIC TECHNIQUES OF NONLINEAR DYNAMICS DISTINGUISH PERIODIC, RANDOM, AND CHAOTIC SIGNALS?' * page 244, line 17 - line 21 * | 1-3 | G06F |
| A | * page 247, line 6 - line 13 * * page 254, line 7 - line 13 * * page 259, line 12 - line 19 * * page 259, line 34 - line 46 * | 4-7 | |

-/--

The present search report has been drawn up for all claims

| Place of search | Date of completion of the search | Examiner |
|---|---|---|
| THE HAGUE | 28 SEPTEMBER 1993 | BARBA M. |

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | US-A-5 201 321 (K.W.FULTON) 13 April 1993 * column 3, line 20 - line 26 * * column 5, line 38 - line 44 * * column 7, line 64 - line 67 * * column 8, line 25 - line 30 * * column 10, line 40 - line 46 * * column 11, line 20 - line 60 * | 1-10 | |
| A | EUROPHYSICS LETTERS, EUROPEAN PHYSICAL SOCIETY vol. 9, no. 3, 1 June 1989, SWITZERLAND pages 191 - 196 M.BAUER ET AL 'EXPERIMENTAL STUDIES OF MODE-LOCKING AND CIRCLE MAPS IN INDUCTIVELY SHUNTED JOSEPHSON JUNCTIONS' * page 193, line 18 - line 27 * | 6 | |
| D,A | AMERICAN HEART JOURNAL, MOSBY-YEAR BOOK, INC. vol. 123, no. 3, March 1992, ST.LOUIS, US pages 704 - 710 M.A.WOO ET AL 'PATTERNS OF BEAT TO BEAT HEART RATE VARIABILITY IN ADVANCED HEART FAILURE' * page 704, left column, line 13 - right column, line 3 * * page 704, right column, line 10 - line 24 * * page 705, right column, line 14 - line 20 * | 1 | TECHNICAL FIELDS SEARCHED (Int. Cl.5 ) |

-/--

The present search report has been drawn up for all claims

| Place of search | Date of completion of the search | Examiner |
|---|---|---|
| THE HAGUE | 28 SEPTEMBER 1993 | BARBA M. |

EPO FORM 1503 03.82 (P0401)

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | BIOLOGICAL CYBERNETICS, SPRINGER INTERNATIONAL<br>vol. 58, 1988,<br>pages 203 - 211<br>A.BABLOYANTZ ET AL 'IS THE NORMAL HEART A PERIODIC OSCILLATOR'<br>* the whole document * | 1-10 | |

TECHNICAL FIELDS
SEARCHED (Int. Cl.5 )

The present search report has been drawn up for all claims

| Place of search | Date of completion of the search | Examiner |
|---|---|---|
| THE HAGUE | 28 SEPTEMBER 1993 | BARBA M. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P0401)